Europäisches Patentamt

European Patent Office

Office européen des brevets

(19)

(11) Numéro de publication: **0 366 550**
**A1**

(12)
# DEMANDE DE BREVET EUROPEEN

(21) Numéro de dépôt: 89402949.5

(22) Date de dépôt: 25.10.89

(51) Int. Cl.5: **C12N 15/31 , C12N 9/88 ,**
**A61K 39/07 , C12Q 1/68 ,**
**A61K 39/10 , A61K 39/40 ,**
**C12P 21/08**

(30) Priorité: 25.10.88 FR 8813952

(43) Date de publication de la demande:
02.05.90 Bulletin 90/18

(84) Etats contractants désignés:
AT BE CH DE ES GB GR IT LI LU NL SE

(71) Demandeur: INSTITUT PASTEUR
25-28, rue du Docteur Roux
F-75724 Paris Cédex 15(FR)

(72) Inventeur: Escuyer, Vincent
5 avenue Blanche de Castille
F-78300 Poissy(FR)
Inventeur: Duflot, Edith
18 rue Pascal
F-94230 Cachan(FR)
Inventeur: Mock, Michèle
6 rue du Cdt Lamy
F-75011 Paris(FR)
Inventeur: Danchin, Antoine
60 rue de Reuilly
F-75012 Paris(FR)

(74) Mandataire: Peaucelle, Chantal et al
S.C. Ernest Gutmann - Yves Plasseraud 67,
boulevard Haussmann
F-75008 Paris(FR)

(54) Séquences de nucléotides exprimant l'adényl cyclase de B.anthracis, protéines ayant l'activité de cette adényl cyclase et applications biologiques.

(57) Les séquences de nucléotides comprennent tout ou partie d'une séquence codant pour une adényl cyclase telle qu'exprimée par B.anthracis.
La protéine exprimée est utilisable pour l'élaboration de vaccins moléculaires à effet protecteurs vis-à-vis des infections dues à B.anthracis et le cas échéant B.pertussis chez l'homme et l'animal.

EP 0 366 550 A1

# SEQUENCES DE NUCLEOTIDES EXPRIMANT L'ADENYL CYCLASE DE B.ANTHRACIS, PROTEINES AYANT L'ACTIVITE DE CETTE ADENYL CYCLASE ET APPLICATIONS BIOLOGIQUES.

L'invention a pour objet des séquences de nucléotides codant pour une adényl cyclase telle qu'exprimée par Bacillus anthracis. Elle vise également les protéines correspondant à cette adényl cyclase et leurs applications biologiques.

B.anthracis est une bactérie gram positif fortement pathogène pour l'homme et l'animal. Sa virulence résulte de la production d'une exotoxine à trois composants et d'une capsule d'acide poly-D-glutamique.

Les trois protéines consistent en un antigène protecteur (PA) de 85 kDa, un facteur léthal (LF) de 83 kDa et un facteur oedèmatogène (EF) de 89 kDa.

Ces protéines ne sont pas toxiques en elles-mêmes. Mais leur interaction provoque deux réponses pathologiques différentes chez l'animal.

Ainsi l'injection de PA avec LF provoque la mort tandis que l'injection intradermique de PA avec EF produit un oedème de la peau chez le cochon d'inde ou le lapin. Le composant EF qui est doté d'une activité adényl cyclase dépendante de la calmoduline, activateur eucaryote, induit une importante augmentation de la concentration en cAMP intracellulaire (AMP cyclique). On sait qu'une autre bactérie pathogène, à savoir Bordetella pertussis, produit également une adényl cyclase toxique, extracellulaire, activée par la calmoduline de l'hôte. Comme l'ont montré Mock et al (1), ces deux adényl cyclase sont antigéniquement reliées bien qu'elles soient produites par des organismes taxonomiquement totalement distincts.

Les gènes responsables de l'expression de PA, LF et EF sont présents sur le plasmide pX01 de B.anthracis. Leur clonage moléculaire et leur expression chez E.coli a été rapportée par Vodkin et Leppla (2), pour PA, Robertson et Leppla (3) pour LF, et Tippetts et Robertson, (4) pour EF.

Mock et al (1) ont également rapporté un procédé de clonage et d'expression de l'adényl cyclase de B.anthracis dans E.coli. Le procédé général de clonage fait l'objet de la demande FR 87/10614 du 24 juillet 1987 aux noms des demandeurs. Brièvement, on utilise selon ce procédé l'interaction adényl cyclase-calmoduline qui se traduit par la production de cAMP. Le clonage du gène est effectué dans une souche réceptrice déficiente en adényl cyclase, portant un plasmide exprimant de hauts niveaux de calmoduline. Les gènes qui coopèrent dans le procédé de clonage, à savoir celui qui code pour l'adényl cyclase et celui qui code pour la calmoduline, sont d'origine différentes

En poursuivant leurs travaux dans ce domaine, les inventeurs ont réussi à déterminer la séquence portant l'information requise pour l'expression d'au moins la partie active d'une adényl cyclase telle qu'exprimée par B.anthracis.

Cette étape a permis de définir la structure du gène, ses particularités ainsi que les similitudes éventuelles avec d'autres gènes. La conduite de ces travaux a également permis de déterminer la structure primaire de la toxine exprimée et de la comparer à la structure de toxines similaires telles que sécrétées par exemple par B.pertussis

L'invention a donc pour but de fournir de nouvelles séquences de nucléotides capables de coder pour des protéines à activité adényl cyclase telle qu'exprimée par B.anthracis.

Elle a également pour but de fournir au moins la partie active, par rapport à une activité adényl cyclase, de ces protéines.

L'invention vise en outre à fournir des vaccins moléculaires renfermant tout ou partie de séquences immunoprotectrices de l'adényl cyclase.

La séquence de nucléotides selon l'invention est caractérisée en ce qu'elle est constituée par au moins une partie d'une séquence codant pour une protéine à activité adényl cyclase telle qu'exprimée par B.anthracis.

Cette séquence est capable de s'hybrider avec des gènes codant pour une protéine à activité adényl cyclase de B.anthracis.

L'invention concerne également une séquence de nucléotides caractérisée en re qu'elle porte l'information pour l'expression d'une adényl cyclase, ou de fragments de cette dernière, capables de former un complexe immunologique avec des anticorps dirigés respectivement contre une adényl cyclase de B.anthracis, de B.pertussis ou de cerveau de rat ou contre des fragments d'une telle adényl cyclase.

L'invention concerne également une séquence recombinante comprenant l'une des séquences définies ci-dessus, le cas échéant associée à un promoteur capable de contrôler la transcription de la séquence d'ADN codant pour des séquences de terminaison de la transcription et des signaux de traduction et de sécrétion.

Elle vise encore une séquence de nucléotides recombinante, associée à un promoteur et à un opérateur permettant de contrôler la transcription, et à une séquence signal permettant la sécrétion de la protéine dans l'espace périplasmique (gram -) ou dans le milieu extérieur.

Selon encore un autre aspect, la séquence de nucléotides de l'invention est capable de s'hybrider avec une sonde formée à partir de la séquence présentant l'enchaînement de nucléotides (I) suivant, qui corresond dans sa totalité au gène cya de B.anthracis :

```
GAATTCAAAATCGGACTTAGAAATACACATATAGAAATAAACAACCTAATCCATGTCACT
                          -400
GTACCGTTTTTTTACTAAATAAACGAAATCAGTGTAAAATGAACAGCTGAACTTTATCA
ACTTAGAATCTCTTTTTTTACTTTAAATGCCTAGCTGTTTTTTCTAATGTTTGTATTTCT
-300
AAATATATTTAAATATGAATTGTAGCTGTGTGCCAAGAGTTATAATTAATTTAAATAAGA
TTATATTTGTAAATAAAATTGTAATTTAACATGTAGAATAAAGAGATTTTTAGTTTTATT
                                       -200
AACAGGATGAAAATCCATAAAACCGTAAATGTGATTTCTAAATTAGTTTAAAATAAAAA
CAAGGATTTGCTCAGACTTGAGATGAATATCTAAATATCAAGACCCAAAGGAGGTTAA
                  -100
GAATGACTAGAAATAAATTTATAACTAATAAGTTTAGTATTATATCCTTTTCAGTATTAC
     20
TATTTGCTATATCCTCCTCACAGGCTATAGAAGTAAATGCTATGAATGAACATTACACTG
                                         100
AGAGTGATATTAAAGAAACCATAAAACTGAAAAATAAACTGAAAAGAAAATTTA
   60
AAGACAGTATTAATAACTTAGTTAAACAGAATTTACCAATGAAACTTTAGATAAAATAC
   200
AGCAGACACAAGACTTATTAAAAAAGATACCTAAGGATGTACTTGAAATTTATAGTGAAT
   100
TAGGAGGAGAAATCTATTTTACAGATATAGATTTAGTAGAACATAAGGAGTTACAAGATT
   300
TAAGTGAAGAAGAGAAAAATAGTATGAATAGTAGAGGTGAAAAGTTCCGTTTGCATCCC
                                         400
GTTTTGTATTTGAAAGAAAGGGAAACACCTAAATTAATTATAAATATCAAGATTATT
   160
CAATTAATAGTGAACAAAGTAAAGAAGTATATTATGAAATTGGAAGGGGATTTCTCTT
                  500
ATATTATAAGTAAGGATAAATCTCTAGATCCAGAGTTTTTAAATTTAATTAAGAGTTTA
   200
GCGATGATAGTGATAGTAGCGACCTTTTATTTAGTCAAAAATTTAAAGAGAAGCTAGAA
   600
```

I b

220
TGAATAATAAAAGTATAGATATAAATTTTATAAAAGAAAATTTAACTGAATTTCAGCATG
700

240
CGTTTTCTTTAGCGTTTTCTTATTATTTTGCACCTGACCATAGAACGGTATTAGAGTTAT

260
ATGCCCCCGACATGTTTGAGTATATGAATAAGTTAGAAAAGCGGGATTTGAGAAAATAA
800

280
GTGAAAGTTTGAAGAAAGAAGGTGTGGAAAAGATAGGATTCATGTGCTGAAAGGAGAAA

300
AAGCACTTAAAGCTTCAGGTTTAGTACCAGAACATGCAGATGCTTTTAAAAAAATTGCTA
900

320
GAGAATTAAATACATATATTCTTTTTAGGCCTGTTAATAAGTTAGCTACAAACCTTATTA
1000

340
AAAGTGGTGTGGCTACAAAGGGATTGAATGTTCATGGAAAGAGTTCGGATTGGGGCCCTG

360
TAGCTGGATACATACCATTTGATCAAGATTTATCTAAGAAGCATGGTCAACAATTAGCTG
1100

380
TCGAGAAAGGAAATTTAGAAAATAAAAATCAATTACAGAGCATGAAGGTGAAATAGGTA

400
AAATACCATTAAAGTTAGACCATTTAAGAATAGAAGAGTTAAAGGAAATGGGATAATTT
1200

420
TGAAGGGTAAAAAGAAATTGATAATGGTAAAAAATATTATTTGTTAGAATCGAATAATC
1300

440
AGGTATATGAATTTAGAATTAGCGATGAAAACAACGAAGTACAATACAAGACAAAAGAAG

460
GTAAAATTACTGTTTTAGGGGAAAAATTCAATTGGAGAAATATAGAAGTGATGGCTAAAA
1400

480
ATGTAGAAGGGGTCTTGAAGCCGTTAACAGCTGACTATGATTTATTTGCACTTGCCCCAA

500
GTTTAACAGAAATAAAAAAACAAATACCACAAAAAGAATGGATAAAGTAGTTAACACCC
1500

4

1 c

```
520
CAAATTCATTAGAAAAGCAAAAAGGTGTTACTAATTTATTCATTAAATATGGAATTGAGA
                                                        1600
540
GGAAACCGGATTCAACTAAGGGAACTTTATCAAATTGGCAAAACAAATGCTTGATCGTT
560
TGAATGAAGCAGTCAAATATACAGGATATACAGGGGGGGGATGTGGTTAACCATGGCACAG
                                                1700
580
AGCAAGATAATGAAGAGTTTCCTGAAAAGATAACGAAATTTTTATAATTAATCCAGAAG
600
GTGAATTTATATTAACTAAAAATTGGGAGATGACAGGTAGATTTATAGAAAAAACATTA
1800
620
CGGGAAAAGATTATTTATATTATTTTAACCGTTCTTATAATAAAATAGCTCCTGGTAATA
                                                        1900
640
AAGCTTATATTGAGTGGACTGATCCGATTACAAAGCCAAATAAATACCATCCCTACGT
660
CAGCAGAGTTTATAAAAAACTTATCCAGTATCAGAAGATCTTCAAATGTAGGAGTTTATA
                                            2000
680
AAGATAGTGGCGACAAAGACGAATTGCAAAAAAGAAAGCGTGAAAAAAATTGCAGGAT
700
ATTTGTCAGACTATTACAATTCAGCAAATCATATTTTTTCTCAGGAAAAAAGCGTAAAA
2100
720
TATCAATATTTCGTGGAATCCAAGCCTATAATGAAATTGAAATGTTCTAAAATCTAAAC
                                                        2200
740
AAATAGCACCAGAATACAAAAATTATTTTCAATATTTAAAGGAAAGGATTACCAATCAAC
760
TTCAATTGCTTCTAACACATCAAAAATCTAATATTGAATTAAATTATTCTATAAACAAT
                                        2300
780
TAAACTTTACAGAAAATGAAACGGATAATTTTGAGGTCTTCCAAAAAATTATTGATGAA
800
AATAAATATATATAATTGTTTTTCTGAAAATTCATCATTTTAAAGAAGACACTAGGAATT
2400
AAATAGATGTATTGAATAGTTATAGTAATGGTCTTGTATCGACATACCGCTTATACTTTT
                                                    2500
GGAGGTAGTAGATATTAAACAACATATAGCAAATGAACTGGATGTAGATC
```

ou l'enchaînement des nucléotides complémentaires de la séquence (I).

Des séquences selon l'invention sont caractérisées en ce qu'elles comprennent l'enchaînement (I) défini ci-dessus ou sont constituées par tout ou partie de cet enchaînement.

La séquence en aval du site de liaison aux ribosomes est caractérisée en ce qu'elle est particulièrement riche en A-T.

Il va de soi que les bases de la séquence de nucléotides considérée peuvent être dans un ordre différent de celui trouvé dans les gènes et/ou que ces bases peuvent être, le cas échéant, substituées, dès lors qu'une sonde élaborée à partir d'une telle séquence donne une réponse caractéristique et non équivoque quant à la capacité de reconnaître la présence de gènes codant pour une adényl cyclase telle que secrétée par B.anthracis.

Toute séquence de nucléotides hybridable avec celle de l'enchaînement (I), telle qu'obtenue par transcription enzymatique inverse de l'ARN correspondant ou encore par synthèse chimique, entre également dans le cadre de l'invention.

La séquence de nucléotides de l'invention correspond encore, selon le code génétique universel, à au moins une partie de la séquence (II) en acides aminés suivantes :


II a


```
      M  T  R  N  K  P  I- P  K  K  F  S  I  I  S  P  S  V  L

 20 L P  A  I  S  S  S  Q  A ? ?  E  V  N  A  N  N  E  N  Y  T

 40 E S  D  I  K  R  N  N  K  T  E  K  N  K  T  E  K  E  K  P

 60 K D  S  I  N  N  L  V  K  T  E  P  T  N  E  T  L  D  K  I

 80 Q Q  T  Q  D  L  L  K  K  I  P  K  D  V  L  E  I  Y  S  E

100 L G  G  E  I  T  P  T  D  I  D  L  V  E  N  K  E  L  Q  D

120 L S  E  E  E  K  N  S  N  N  S  R  G  E  K  V  P  P  A  S

140 R P  V  P  E  K  K  R  E  T  P  K  L  I  I  N  I  K  D  T

160 A I  N  S  E  Q  S  K  E  V  T  T  E  I  G  K  G  I  S  L

180 D I  I  S  K  D  K  S  L  D  P  E  P  L  N  L  I  K  S  L

200 S D  D  S  D  S  S  D  L  L  P  S  Q  K  P  K  E  K  L  E
```

II b

```
220 L  M  M  X  S  X  D  X  M  F  X  K  X  M  L  T  X  F  Q  M
240 A  F  S  L  A  F  S  Y  Y  F  A  P  D  X  R  T  V  L  X  L
260 Y  A  F  D  K  X  X  X  K  N  K  L  X  X  G  G  F  X  K  X
280 S  X  S  L  X  X  X  G  V  X  X  D  R  X  D  V  L  K  G  X
300 K  A  L  X  A  S  G  L  V  P  X  M  A  D  A  F  K  K  X  A
320 R  X  L  M  T  T  X  L  F  K  F  V  M  X  L  A  T  M  L  X
340 X  S  G  V  A  T  X  G  L  M  V  M  G  X  S  S  D  W  G  F
360 V  A  G  Y  X  P  F  D  Q  D  L  S  X  X  M  G  Q  Q  L  A
380 V  X  K  G  M  L  X  M  X  X  S  X  T  X  M  X  G  X  X  G
400 X  X  F  L  X  L  D  X  L  X  X  X  X  L  X  X  M  G  X  X
420 L  K  G  X  X  X  X  D  M  G  X  X  Y  Y  L  L  X  S  M  M
440 Q  V  Y  X  F  R  X  S  D  X  M  M  X  V  Q  Y  K  T  K  X
460 G  K  X  T  V  L  G  X  K  F  N  M  R  M  X  X  V  M  A  K
480 M  V  X  G  V  L  X  F  L  T  A  D  Y  D  L  F  A  L  A  F
500 S  L  T  X  X  X  X  Q  X  F  Q  X  X  M  D  X  V  V  M  T
```

II c

520 P N S L E K Q K G V T N L L I K Y G I E

540 R K P D S T K G T L S N W Q K Q N L D R

560 L N E A V K Y T G Y T G C D V V N H G T

580 E Q D N E S F P E K D N E I P I T N P . E

600 G E F I L T K N W E H T G R F I E K N I

620 T G K D Y L Y Y F N R S Y N K I A P G N

640 K A Y I E W T D P I T K A K I N T I P T

660 S A E F I K N L S S I R R S S N V G V Y

680 K D S G D K D E F A K K E S V K K I A G

700 Y L S D Y Y N S A N H I F S Q E K K R K

720 I S I F R G I Q A Y N E I E N V L K S K

740 Q I A F E Y K N Y F Q Y L K E R I T N Q

760 V Q L L L T H Q K S N I E F K L L Y K Q

780 L N F T E N E T D N F E V F Q K I I D E

800 K •••

Les lettres indiquées dans cet enchaînement présentent les significations conventionnelles suivantes :
D Acide aspartique
E Acide glutamique
A Alanine
R Arginine
N Asparagine
C Cystéine

8

Q Glutamine
G Glycine
H Histidine
I Isoleucine
L Leucine
K Lysine
M Méthionine
F Phenylalanine
P Proline
S Sérine
T Thréonine
W Tryptophane
Y Tyrosine
V Valine

L'invention vise en outre une protéine à activité adényl cyclase du type de celle synthétisée par B.anthracis, ainsi que les fragments peptidiques de cette protéine, correspondant selon le code génétique universel à l'une des séquences de nucléotides ci-dessus.

La protéine selon l'invention est telle qu'obtenue par transformation de cellules hôtes au moyen d'un vecteur recombinant contenant une séquence de nucléotides comme définie plus haut sous le contrôle d'éléments de régulation permettant l'expression de ladite séquence dans la cellule hôte, mise en culture dans un milieu approprié des cellules hôtes transformées et récupération de la protéine à partir de ces cellules ou directement à partir du milieu de culture lorsqu'elle est sécrétée.

L'invention vise plus spécialement une protéine correspondant, selon le code génétique universel, à la séquence (I) de nucléotides et qui est représentée par l'enchaînement (II) de 800 acides aminés. Cette protéine possède un poids moléculaire théorique $M_r$ de 92 387. Comme d'autres protéines extracellulaires d'organismes gram positifs, l'adényl cyclase apparaît synthétisée par B.anthracis sous forme d'un plus grand précurseur dont la séquence signal est éliminée lors de la secrétion. Le début de la séquence devrait occuper la position 29 ou pourrait occuper la position 34. La composition globale après élimination de la séquence du précurseur correspond à une protéine hydrophile. Des résidus basiques ainsi que des acides aminés hydrophobes sont proches de la partie N-terminale comme caractéristiques d'un peptide signal. On peut substituer à cet enchaînement signal d'autres peptides signaux, en particulier pour améliorer la sécrétion de l'adényl cyclase dans d'autres organismes.

On notera que l'adényl cyclase ci-dessus, comme celle exprimée par B.pertussis, ne contient pas de résidus cystéine, ce qui contraste avec les observations biochimiques effectuées sur les cyclases eucaryotes.

L'étude de cette séquence de 800 acides aminés montre qu'elle présente plusieurs régions communes avec l'adényl cyclase sécrétée par B.pertussis de 1706 acides aminés, comme développé dans la partie de la description illustrant plus en détail l'invention.

Selon un autre aspect de l'invention, la protéine de l'invention à activité adényl cyclase donne lieu à une réaction immunologique croisée avec des anticorps dirigés contre des sous-unités catalytiques d'adényl cyclase de cerveau de rat ou encore contre l'adényl cyclase de B.pertussis.

La protéine de l'invention et ses fragments, qui peuvent être également obtenus par synthèse chimique, présentent avantageusement un degré de pureté élevé et sont utilisés pour former, selon les techniques classiques, des anticorps polyclonaux.

De tels anticorps polyclonaux, ainsi que les anticorps monoclonaux capables de reconnaître spécifiquement la protéine ci-dessus et ses fragments sont également visés par l'invention.

Les séquences de nucléotides selon l'invention sont obtenues avantageusement selon le procédé de clonage des demandeurs évoqué plus haut. Les vecteurs recombinants d'expression et de clonage capables de transformer une cellule hôte appropriée entrent également dans le cadre de l'invention. Ces vecteurs comportent au moins une partie d'une séquence de nucléotides de l'invention sous le contrôle d'éléments de régulation permettant son expression. Les souches de microorganismes transformées font également partie de l'invention. Ces souches comportent l'une des séquences de nucleotides définies ci-dessus ou encore un vecteur recombinant tel que défini précedemment.

L'invention vise également les applications biologiques des séquences de nucléotides, des protéines correspondantes et des anticorps monoclonaux ou polyclonaux.

Ces applications comprennent l'élaboration, à partir de fragments intragéniques de l'enchaînement (I), de sondes pour la détection de séquences similaires dans les gènes produisant des adényl cyclases. Cette élaboration comprend, notamment, la dénaturation des séquences double brin pour obtenir une séquence monobrin utilisable en tant que sonde

L'invention vise donc des sondes de détection caractérisées en ce qu'elles comprennent au moins une partie d'une séquence de nucléotides définie ci-dessus, plus spécialement un fragment intragénique codant pour le site catalytique de liaison à la calmoduline ou de manière suffisamment spécifique pour une partie de ce site, dans les adényl cyclases bactérienne. De tels fragments, complémentaires des sites corespondants dans les gènes d'eucaryotes exprimant de l'adényl cyclase à activité dépendante de la calmoduline, présentent en particulier l'avantage de faciliter le clonage et l'analyse des gènes en question.

Le fragment d'ADN utilisé comme sonde comporte un nombre de nucléotides suffisant pour obtenir la spécificité requise et la formation d'un hybride stable.

Des sondes appropriées pour ce type de détection sont avantageusement marquées par un élément radio-actif (sondes chaudes) ou tout autre groupe non radio-actif (sondes froides) permettant la reconnaissance de la sonde à l'état hybridé avec la préparation renfermant l'ADN à étudier.

Selon les techniques classiques, ces sondes sont mises en contact avec un échantillon biologique renfermant des bactéries, ou directement avec ces bactéries ou leurs acides nucléiques, dans des conditions autorisant l'hybridation éventuelle de la séquence de nucléotides de la sonde avec une séquence complémentaire, éventuellement contenue dans le produit testé.

On peut, par exemple, mettre en oeuvre la méthode d'hybridation sur taches. Cette méthode comporte après dénaturation de l'ADN préalablement obtenu à partir de cellules exprimant de l'adényl cyclase, le dépôt d'une quantité aliquote de cet ADN sur des membranes de nitrocellulose, l'hybridation de chaque membrane dans les conditions usuelles avec la sonde et la détection, de manière classique, de l'hybride formé.

On peut aussi utiliser une méthode d'hybridation sur réplique, selon la technique de Southern. Cette méthode comprend la séparation électrophorétique en gel d'agarose des fragments d'ADNs engendrés après traitement de l'ADNs par des enzymes de restriction, le transfert après dénaturation alcaline sur des membranes appropriées et leur hybridation avec la sonde dans les conditions usuelles. Il n'est pas toujours nécessaire de procéder à l'expression préalable de l'ADN. Il suffit que l'ADN soit rendu accessible à la sonde.

L'invention fournit ainsi des outils permettant de détecter rapidement, avec une grande spécificité, des séquences similaires dans les gènes codant pour des adényl cyclases, ce qui permet d'étudier l'origine et le mode d'action de ces adényl cyclases.

Pour la mise en oeuvre des méthodes de détection considérées ci-dessus basées sur l'utilisation de sondes nucléotidiques, on a recours avantageusement à des nécessaires ou kits comprenant :
- une quantité déterminée d'une sonde nucléotidique selon l'invention,
- avantageusement, un milieu approprié respectivement à la formation d'une réaction d'hybridation entre la séquence à détecter et la sonde,
- avantageusement des réactifs permettant la détection des complexes d'hybridation formés entre la séquence de nucléotides et la sonde lors de la réaction d'hybridation.

L'invention vise également les applic tions immunologiques des protéines définies ci-dessus, plus spécialement pour l'élaboration d'antisérums spécifiques ainsi que d'anticorps polyclonaux et monoclonaux. Les anticorps polyclonaux sont formés selon les techniques classiques par injection de la protéine à des animaux, récupération des antisérums, puis des anticorps à partir des antisérums par exemple par chromatographie d'affinité.

Les anticorps monoclonaux sont produits de manière habituelle en fusionnant des cellules de myélomes avec des cellules de rate d'animaux préalablement immunisés à l'aide des protéines de l'invention.

Les essais de toxicologie réalisés avec l'adényl cyclase en l'absence d'antigène protecteur ont démontré leur absence de toxicité.

Tout ou partie des séquences immunoprotectrices de ces protéines sont avantageusement utilisées pour l'élaboration de vaccins sous réserve de ne pas donner lieu à des réactions immunitaires indésirables mettant en jeu par exemple l'adényl cyclase de l'hôte.

L'invention vise donc des vaccins moléculaires capables de prévenir les infections provoquées par B.anthracis et ses effets toxiques chez l'homme et l'animal, ces vaccins étant à base des protéines définies ci-dessus, avec un véhicule pharmaceutique.

Les anticorps formés contre l'adényl cyclase de B.anthracis donnant lieu une réaction immunologique croisée avec l'adényl cyclase de B.pertussis, l'invention fournit avantageusement un double vaccin contre les infections provoquées par B.anthracis et B.pertussis.

On indique ci-après, à titre d'exemple non limitatif, les matériels et les méthodes utilisés pour cloner et séquencer le gène de l'adényl cyclase de B.anthracis.

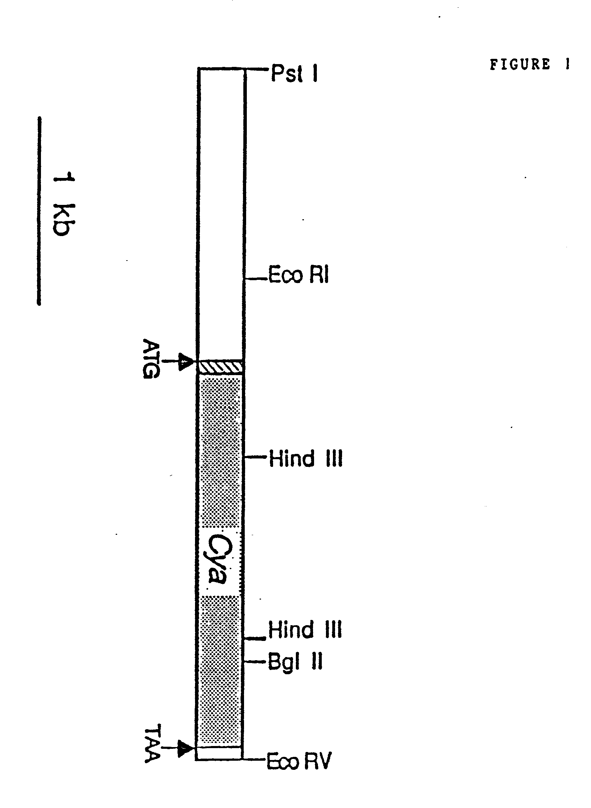

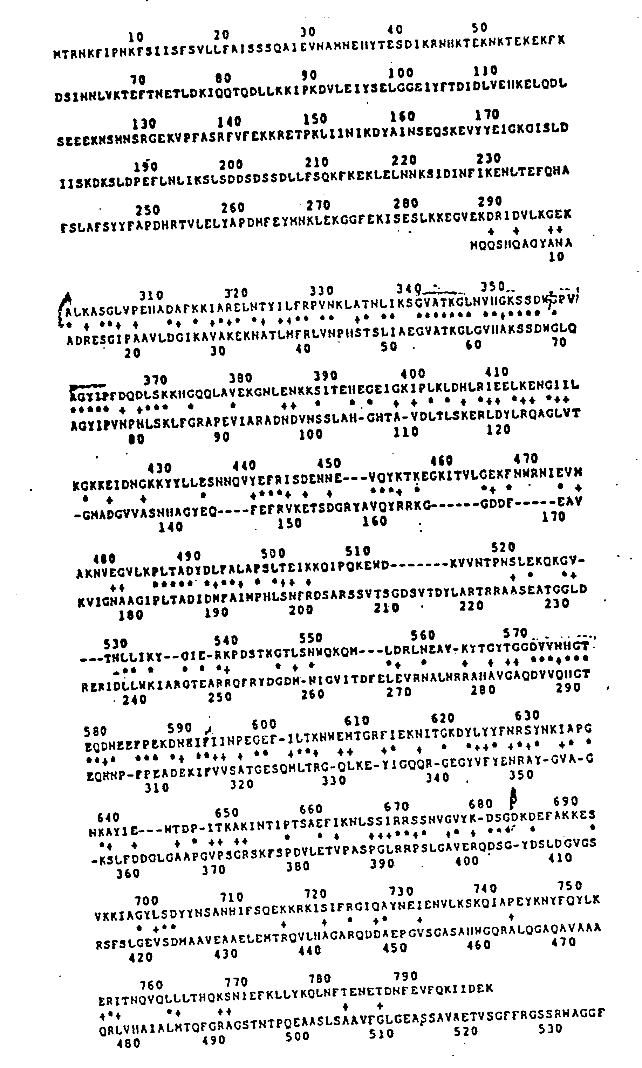

Les figures 1 et 2 auxquelles il est fait référence représentent :

- la figure 1, la carte de restriction d'un fragment d'ADN d'environ 3,8 kb portant le gène cya de B.anthracis, et
- la figure 2, les séquences d'acides aminés des adényl cyclases de B.anthracis (ligne supérieure) et de B.pertussis (ligne inférieure).

## a) Souches bactériennes et plasmides

On utilise les souches d'E.coli suivantes pour la transformation TP610 (5) et pour la transfection JM105 (6).

le plasmide recombinant mis en oeuvre pMMA8812 est un dérivé de pUC8 contenant un fragment EcoRV-PstI de 3,8 kb portant les déterminants pour l'adénylcyclase de B.anthracis (1). Le fragment de restriction EcoRV-PstI est représenté sur la Figure 1.

Le plasmide recombinant pMMA8812 exerce une action de complémentation, activée par la calmoduline, vis-à-vis de la déficience en cyclase d'une souche d'E.coli cya⁻.

## b) Milieux et réactifs chimiques

On réalise les cultures sur des milieux LB riches de Miller (7). L ampicilline, lorsqu'elle est utilisée, est ajoutée à raison de 100 microgramme par ml. On utilise les enzymes de restriction ADN T4 ligase et PolIk (Boehringer - Mannheim) et l'ADN T7 polymérase modifiée (Séquenase) commercialisée par USBC. Les oligodéoxy ribonucléotides utilisés comme amorces dans le séquençage d'ADN d'une part et le dATP³⁵ d'autre part sont tels que commercialisés respectivement par Pharma et par Amersham.

## c) Analyse de la séquence de nucléotides

On utilise des sous-clones dans le vecteur mono-brin M13mp19 (8). Pour engendrer des délétions unidirectionnelles, on a recours au système cyclone (IBI).

La séquence de nucléotides est déterminée selon la méthode de terminaison de chaine didéoxynucléotide (9) lorsqu'on utilise PolIk ou par une méthode de terminaison de chaîne didéoxynucléotide modifiée, lorsqu'on utilise la Séquenase (10).

On a déterminé la séquence de nucléotides de l'insertion d'ADN de B.anthracis de pMMA8812.

Le fragment de la figure 1 ne comporte qu'un cadre ouvert de lecture de 2400 pb. Ce dernier contient 800 codons allant du site considéré comme étant le site d'initiation de la traduction au codon de terminaison TAA.

Le codon considéré comme codon ATG de départ est précédé par un site, du type des sites de liaison aux ribosomes (RBS), dont la séquence GGAGG est complémentaire du 3'OH de l'ARN ribosomal 16S.

La séquence complète de nucléotides du cadre ouvert de lecture est représentée sur la figure 2. On constate que la séquence en aval du site RBS est particulièrement riche en A-T. La séquence d'acides aminés traduite correspond à l'enchaînement II ci-dessus.

Le site de clivage proposé sur la Figure 2 doit être corroboré par des données concernant le résidu N-terminal de l'adényl cyclase. Comme indiqué plus haut, l'adényl cyclase est vraisemblablement synthétisée par B.anthracis sous forme d'un plus grand précurseur dont la séquence signal est éliminée lors de la sécrétion. Le traitement du précurseur devrait conduire à une protéine mature de $M_r$ 89261.

## d) Comparaison des structures primaires des adényl-cyclases de B.anthracis et de B.pertussis

On a représenté sur la figure 2, la séquence complète du polypeptide de l'adényl cyclase de B.anthracis (ligne supérieure) et la séquence polypeptidique N-terminale de l'adenyl cyclase de B.pertussis (ligne inférieure).

Les astérisques indiquent les acides aminés identiques et les croix des acides aminés de la même classe chimique.

La comparaison de ces séquences montre qu'elles sont dans leur ensemble différentes mais qu'elles présentent des parties de forte ou de plus faible similitude

On constate, en particulier, une similitude dans un domaine d'environ 400 acides aminés situé chez l'enzyme de B.anthracis dans la partie centrale et chez celle de B.pertussis dans la partie N-terminale.

Des expériences de délétion in vitro ont montré que la séquence de 450 acides aminés de l'extrémité N-terminale de l'adényl cyclase de B.pertussis correspond au domaine catalytique activé par la calmoduline, ce qui amène à reconnaître le centre catalytique de l'adényl cyclase de B.anthracis dans la région allant de l'acide aminé en position 300 à celui en position 683.

La similitude la plus importante correspond au peptide de 24 acides aminés (de la position 342 à la position 365). Cette séquence contient cinq résidus gly et la séquence noyau G --- GKS (AKS chez B.pertussis) que l'on retrouve souvent dans les protéines ayant une affinité pour les nucléotides.

Deux autres régions présentent une similitude plus faible. Elles correspondent aux domaines s'étendant des positions 487 à 501 d'une part et 573 à 594 d'autre part.

Selon les résultats des études effectuées sur cette séquence d'acides aminés l'adényl cyclase de B.anthracis apparait organisée en domaines fonctionnels.

Au moins trois fonctions peuvent être attribuées à la molécule, à savoir :

1 - l'interaction avec des cellules eucaryotes. Cette propriété s'exerce par l'intermédiaire de l'antigène protecteur (PA) qui se fixe tout d'abord aux cellules sensibles, puis interagit avec l'adényl cyclase,

2 - l'internalisation,

3 - la fixation à la calmoduline et l'activation de la cyclase.

La partie centrale de la molécule (région allant des positions 350 à 390) est attribuée au domaine dépendant de la calmoduline.


## REFERENCES BIBLIOGRAPHIQUES

1) Mock et al, Gene 1988, 64, 277-284

2) Vodkin et Leppla, Cell 1983, 34:693-697

3) Robertson et Leppla, Gene 1986, 44:71-78

4) Tippetts et Robertson, J. of Bact. 1988, vol 70, n° 5, 2263-2266

5) Hedegaard et Danchin, Mol. Gen. Gen. 201 (1985), 38-42

6) Yanisch-Perron et al, Gene 33 (1985), 103-119

7) Miller, Cold Spring harbor, NY, 1972

8) Norrander et al, Gene 26 (1983), 101-106

9) Sanger et al, Proc. Natl. Acad. Sci. USA 74 (1977), 5463-5467

10) Tabor et Richardson, Proc. Natl. Acad. Sci. USA 84 (1987), 4767-4771

**Revendications**

1. Séquence de nucléotides caractérisée en ce qu'elle est constituée par au moins une partie d'une séquence codant pour une protéine à activité adényl cyclase telle qu'exprimée par B.anthracis.

2. Séquence de nucléotides, caractérisée en ce qu'elle est capable de s'hybrider avec des gènes codant pour une adényl cyclase de B.anthracis.

3. Séquence selon la revendication 1 ou 2, caractérisée en ce qu'elle porte l'information pour l'expression d'une adényl cyclase, ou de fragments de cette dernières capables de former un complexe immunologique avec des anticorps dirigés respectivement contre une adényl cyclase de B.anthracis ou de B.pertussis, ou contre des fragments d'une telle adényl cyclases

4. Séquence de nucleotides recombinante, caractérisée en ce qu'elle comprend une séquence selon l'une des revendications 1 à 3, le cas échéant associée à un promoteur capable de contrôler la transcription de la séquence et une séquence d'ADN codant pour des signaux de terminaison de la transcription.

5. Séquence de nucléotides recombinante, associée à un promoteur et à un opérateur permettant de contrôler la transcription, et à une séquence signal permettant la sécrétion de la protéine dans l'espace périplasmique (gram -) ou dans le milieu extérieur.

6. Séquence de nucléotides, caractérisée en ce qu'elle est capable de s'hybrider avec une sonde formée à partir de la séquence présentant l'enchaînement de nucléotides (I) suivant :

I a

GAATTCAAAATCGGACTTAGAAATACACATATAGAAATAAACAACCTAATCCATGTCACT
-400
GTACCGTTTTTTTACTAAATAAACGAAATCAGTGTAAAATGAACAGCTGAACTTTATCA
ACTTAGAATCTCTTTTTTTACTTTAAATGCCTAGCTGTTTTTTCTAATGTTTGTATTTCT
-300
AAATATATTTAAATATGAATTGTAGCTGTGTGCCAAGAGTTATAATTAATTTAAATAAGA
-200
TTATATTTGTAAATAAAATTGTAATTTAACATGTAGAATAAAGAGATTTTTAGTTTTATT
AACAGGATGAAAATCCATAAAACCGTAAATGTGATTTCTAAATTAGTTTAAAATAAAAA
-100
CAAGGATTTGCTCAGACTTGAGATGAATATCTAAATATCAAGAACCCAAAGGAGG TTAA

GAATGACTAGAAATAAATTTATAACTAATAAGTTTAGTATTATATCCTTTTCAGTATTAC

TATTTGCTATATCCTCCTCACAGGCTATAGAAGTAAATGCTATGAATGAACATTACACTG
100
AGAGTGATATTAAAAGAAACCATAAAACTGAAAAAATAAAACTGAAAAGAAAATTTA

AAGACAGTATTAATAACTTAGTTAAAACAGAATTTACCAATGAAACTTTAGATAAAATAC
200
AGCAGACACAAGACTTATTAAAAAAGATACCTAAGGATGTACTTGAAATTTATAGTGAAT

TAGGAGGAGAAATCTATTTTACAGATATAGATTTAGTAGAACATAAGGAGTTACAAGATT
300
TAAGTGAAGAAGAGAAAAATAGTATGAATAGTAGAGGTGAAAAGTTCCGTTTGCATCCC
400
GTTTTGTATTTGAAAAGAAAGGGAAACACCTAAATTAATTATAAATATCAAAGATTATT

CAATTAATAGTGAACAAAGTAAAGAAGTATATTATGAAATTGGAAAGGGGATTTCTCTT
500
ATATTATAAGTAAGGATAAATCTCTAGATCCAGAGTTTTTAAATTTAATTAAGAGTTTA

GCGATGATAGTGATAGTAGCGACCTTTTATTTAGTCAAAAATTTAAAGAGAAGCTAGAA
600

13

I   b

220
TGAATAATAAAGTATAGATATAAATTTTATAAAAGAAAATTTAACTGAATTTCAGCATG
                                                          700
240
CGTTTTCTTTAGCGTTTTCTTATTATTTTGCACCTGACCATAGAACGGTATTAGAGTTAT
260
ATGCCCCCGACATGTTTGAGTATATGAATAAGTTAGAAAAGGGGGATTTGAGAAAATAA
              800
280
GTGAAAGTTTGAAGAAAGAAGGTGTGGAAAAGATAGGATTGATGTGCTGAAAGGAGAAA

300
AAGCACTTAAAGCTTCAGGTTTAGTACCAGAACATGCAGATGCTTTTAAAAAAATTGCTA
900
320
GAGAATTAAATACATATATTCTTTTTAGGCCTGTTAATAAGTTAGCTACAAACCTTATTA
                                              1000
340
AAAGTGGTGTCGCTACAAAGGGATTGAATGTTCATGGAAAGAGTTCGGATTGGGGCCCTG

360
TAGCTGGATACATACCATTTGATCAAGATTTATCTAAGAAGCATGGTCAACAATTAGCTG
            1100
380
TCGAGAAAGGAAATTTAGAAAATAAAAAATCAATTACAGAGCATGAAGGTGAAATAGGTA

400
AAATACCATTAAAGTTAGACCATTTAAGAATAGAAGAGTTAAAGGAAATGGGATAATTT
  1200
420
TGAAGGGTAAAAAGAAATTGATAATGGTAAAAAATATTATTTGTTAGAATCGAATAATC
                                                  1300
440
AGGTATATGAATTTAGAATTAGCGATGAAAACAACGAAGTACAATACAAGACAAAAGAAG

460
GTAAAATTACTGTTTTAGGGGAAAAATTCAATTGGAGAAATATAGAAGTCATGGCTAAAA
            1400
480
ATGTAGAAGGGGTCTTGAAGCCGTTAACAGCTGACTATGATTTATTTGCACTTGCCCCAA

500
GTTTAACAGAAATAAAAAAACAAATACCACAAAAAGAATGGGATAAAGTAGTTAACACCC
1500

1 c

CAAATTCATTAGAAAAGCAAAAGGTGTTACTAATTTATTGATTAAATATGGAATTGAGA
1600

GGAAACCGGATTCAACTAAGGGAACTTTATCAAATTGGCAAAACAAATGCTTGATCGTT

TGAATGAAGCAGTCAAATATACAGGATATACAGGGGGGGATGTGGTTAACCATGGCACAG
1700

AGCAAGATAATGAAGAGTTTCCTGAAAAGATAACGAAATTTTTATAATTAATCCAGAAG

GTGAATTTATATTAACTAAAAATTGGGAGATGACAGGTAGATTTATAGAAAAAACATTA
1800

CGGGAAAAGATTATTTATATTATTTTAACCGTTCTTATAATAAAATAGCTCCTGGTAATA
1900

AAGCTTATATTGAGTGGACTGATCCGATTACAAAAGCCAAATAAATACCATCCCTACGT

CAGCAGAGTTTATAAAAAACTTATCCAGTATCAGAAGATCTTCAAATGTAGGAGTTTATA
2000

AAGATAGTGGCGACAAAGACGAATTTGCAAAAAAGAAAGCGTGAAAAAAATTGCAGGAT

ATTTGTCAGACTATTACAATTCAGCAAATCATATTTTTTCTCAGGAAAAAAAGCGTAAAA
2100

TATCAATATTTCGTGGAATCCAAGCCTATAATGAAATTGAAATGTTCTAAAATCTAAAC
2200

AAATAGCACCAGAATACAAAAATTATTTTCAATATTTAAAGGAAAGGATTACCAATCAAC

TTCAATTGCTTCTAACACATCAAAAATCTAATATTGAATTTAAATTATTCTATAAACAAT
2300

TAAACTTTACAGAAAATGAAACGGATAATTTTGAGGTCTTCCAAAAAATTATTGATGAA

AATAAATATATATAATTGTTTTTTCTGAAAATTCATCATTTTAAAGAAGACACTAGGAATT
2400

AAATAGATGTATTGAATAGTTATAGTAATGGTCTTGTATGGACATACCGCTTATACTTT
2500

GGAGGTAGTAGATATTAAACAACATATAGCAAATGAACTGGATGTAGATC

ou l'enchaînement des nucléotides complémentaires de la séquence (I).

7. Séquence de nucléotides portant l'information génétique correspondant à l'expression d'au moins une partie d'une adényl cyclase telle qu'exprimée par B.anthracis, caractérisée en ce qu'elle comprend au moins une partie de l'enchaînement (I) selon la revendication 6.

8. Séquence de nucléotides, caractérisée en ce qu'elle porte l'information correspondant à l'expression d'au moins une partie de la séquence en acides aminés (II) suivante :

i i a

```
    M T R N K P I P K E P S I I S P S V L
20  L P A I S S S Q A I I E V N A N N E N Y T
40  E S D I K R N N K T E K N K T E K E K P
60  K D S I N N L V K T E P T N E T L D K I
80  Q Q T Q D L L K K I P K D V L E I Y S E
100 L G G E I Y P T D I D L V E N K E L Q D
120 L S E E E K N S N N S R G E K V P P A S
140 R P V P E K R R E T P K L I I N I K D Y
160 A I N S E Q S K E V T Y E I G K G I S L
180 D I I S K D K S L D P E P L N L I K S L
200 S D D S D S S D L L P S Q K P K E K L E
```

II b

```
220 L  N  N  R  S  I  D  I  N  P  I  K  E  N  L  T  E  P  Q  N
240 A  P  S  L  A  P  S  Y  Y  P  A  P  D  N  R  T  V  L  E  L
260 Y  A  P  D  N  E  E  X  N  N  R  L  E  K  G  G  P  E  X  I
280 S  E  S  L  R  R  E  G  V  E  K  D  R  I  D  V  L  K  G  E
300 K  A  L  R  A  S  E  L  V  P  E  N  A  D  A  P  K  K  I  A
320 R  E  L  N  T  T  I  L  P  R  P  V  N  X  L  A  T  N  L  I
340 X  S  G  V  A  T  R  G  L  N  V  H  G  R  S  S  D  W  G  P
360 V  A  G  Y  I  P  P  D  Q  D  L  S  K  I  N  G  Q  Q  L  A
380 V  E  R  G  N  L  E  N  R  R  S  I  T  E  R  E  G  E  I  G
400 X  I  P  L  R  L  Q  N  L  R  I  E  E  L  K  E  N  G  I  I
420 L  K  G  R  R  E  I  D  N  G  R  R  Y  Y  L  L  E  S  N  N
440 Q  V  Y  E  P  R  I  S  D  E  N  N  E  V  Q  Y  K  T  R  E
460 G  R  I  T  V  L  G  E  R  P  N  N  R  N  I  E  V  H  A  R
480 N  V  E  G  V  L  R  P  L  T  A  D  Y  D  L  P  A  L  A  P
500 S  L  T  E  I  R  R  Q  I  P  Q  R  E  N  D  R  V  V  N  T
```

II c

```
520 P N S L E K Q K G V T N L L I K Y G I E
540 R K P D S T K G T L S N W Q K Q M L D R
560 L N E A V K Y T G Y T G G D V V N H G T
580 E Q D N E E F P E K D N E I F I T N P.E
600 G E F I L T K N W E M T G R P I E K N I
620 T G K D Y L Y Y P N R S Y N K I A P G N
640 K A Y I E W T D P I T K A K I N T I P T
660 S A E F I K N L S S I R R S S N V G V Y
680 K D S G D K D E P A K K E S V K K I A G .
700 Y L S D Y Y N S A N H I F S Q E K K R K
720 I S I F R G I Q A Y N E I E N V L K S K
740 Q I A P E Y K N Y F Q Y L K E R I T N Q
760 V Q L L L T H O K S N I E.F K L L Y K Q
780 L N F T E N E T D N F E V F Q K I I D E
800 K •••
```

9. Protéine à activité adènyl cyclase du type de celle exprimée par B.anthracis et ses fragments peptidiques, correspondant selon le code génétique universel, aux séquences de nucléotides de l'une quelconque des revendications 1 à 8.

10. Protéine selon la revendication 9, correspondant à l'enchaînement II d'acides aminés selon la revendication 8.

18

11. Protéine selon la revendication 9 ou 10, caractérisée en ce qu'elle comporte avec l'adényl cyclase de B.pertussis une similitude forte pour les régions allant des positions 342 à 365, et une similitude plus faible pour les domaines s'étendant des positions 487 à 501 d'une part et 573 à 594 d'autre part.

12. Protéine constituée par ou comprenant les domaines allant de la position 300 à la position 683 dans l'enchaînement II selon la revendication 8, correspondant au centre catalytique de l'adényl cyclase de B.pertussis ou au domaine allant de la position 350 à 390 de l'enchaînement II, correspondant au domaine dépendant de la calmoduline.

13. Protéine à activité adényl cyclase selon l'une quelconque des revendications 9 à 12, caractérisée en ce qu'elle donne lieu à une réaction immunologique croisée avec des anticorps dirigés contre l'adényl cyclase de cerveau de rat ou encore l'adényl cyclase de B.pertussis.

14. Anticorps polyclonal ou monoclonal, caractérisé en ce qu'il est dirigé contre tout ou partie de la protéine, ou de ses fragments, selon l'une quelconque des revendications 9 à 13.

15. Sonde de détection caractérisée en ce qu'elle comprend tout ou partie des séquences de nucléotides selon l'une quelconque des revendications 1 à 80.

16. Nécessaire ou kit pour la mise en oeuvre d'une méthode de détection du site de liaison à la calmoduline dans des gènes codant pour une adényl cyclase, caractérisé en qu'il comprend :
- une quantité déterminée d'une sonde nucléotidique selon la revendication 15,
- avantageusement, un milieu approprié à la formation d'une réaction d'hybridation entre la séquence à détecter, et la sonde sus-mentionnée,
- avantageusement, des réactifs permettant la détection des complexes d'hybridation formés entre la séquence de nucléotides et la sonde lors de la réaciton d'hybridation.

17. Vaccins capables d'induire une protection chez l'homme et l'animal contre une infection provoquée par B.anthracis et le cas échéant B.pertussis, caractérisés en ce qu'il s'agit de vaccins moléculaires renfermant une protéine selon l'une quelconque des revendications 9 à 13, en association avec un véhicule pharmaceutique.

FIGURE 1

```
          10             20             30             40             50
MTRNKFIPHKFSIISFSVLLFAISSSQAIEVHAHNEHYTESDIKRHHKTEKHKTEKEKFK

          70             80             90            100            110
DSINHLVKTEFTNETLDKIQQTQDLLKKIPKDVLEIYSELGGEIYFTDIDLVEHKELQDL

         130            140            150            160            170
SEEEKNSHNSRGEKVPFASRFVFEKKRETPKLIINIKDYAINSEQSKEVYYEIGKGISLD

         190            200            210            220            230
IISKDKSLDPEFLHLIKSLSDDSDSSDLLFSQKFKEKLELNHKSIDINFIKEHLTEFQHA

         250            260            270            280            290
FSLAFSYYFAPDHRTVLELYAPDHFEYHNKLEKGGFEKISESLKKEGVEKDRIDVLKGEK
                                                   +   +   ++
                                               HQQSHQAGYANA
                                                         10


        310            320            330            340      350
 ALKASGLVPEHADAFKKIARELNTYILFRPVNKLATHLIKSGVATKGLHVIIGKSSDWSPVI
 +  + ***+  +      ++  + +*+* +  +++* **  ****** **+**** *
 ADRESGIPAAVLDGIKAVAKEKNATLMFRLVNPHSTSLIAEGVATKGLGVIIAKSSDWGLQ
       20             30             40             50        60        70


        370            380            390            400            410
 AGYIPFDQDLSKKHGQQLAVEKGNLENKKSITEHEGEIGKIPLKLDHLRIEELKENGIIL
 ***** + +****  *      ++  *   * *  + *  +  *++ *++ *++
 AGYIPVNPHLSKLFGRAPEVIARADNDVNSSLAH-GHTA-VDLTLSKERLDYLRQAGLVT
       80             90            100            110            120


        430            440            450            460            470
 KGKKEIDNGKKYYLLESNHQVYEFRISDEHNE---VQYKTKEGKITVLGEKFNWRNIEVH
 •  +    +       *      +****+ + +   ***+ +      *+ •   + •  +
 -GHADGVVASNHAGYEQ----FEFRVKETSDGRYAVQYRRKG------GDDF-----EAV
             140            150            160                 170


   480       490            500            510            520
AKNVEGVLKPLTADYDLFALAPSLTEIKKQIPQKEHD-------KVVNTPHSLEKQKGV-
  ++     *****  *+**+ •  *+    • • + •         + •         •• +
KVIGHAAGIPLTADIDMFAIHPHLSNFRDSARSSVTSGDSVTDYLARTRRAASEATGGLD
  180       190            200            210            220        230


        530            540            550            560      570
---THLLIKY--GIE-RKPDSTKGTLSNWQKQH---LDRLHEAV-KITGYTGGDVVNHGT
   •** •    •  •  •+     • • •         •+     +  + ++ ***+***
RERIDLLNKIARGTEARRQFRYDGDH-NIGVITDFELEVRNALHRRAHAVGAQDVVQHGT
   240            250            260            270            280        290


  580       590         600            610            620            630
EQDHEEFPEKDHEIFIINPEGEF-ILTKNWEHTGRFIEKNITGKDYLYYFNRSYHKIAPG
 **+*  *** *+ **++ + **  *+**  ++   + •  +•* +*+* +• •
EQHNP-FPEADEKIFVVSATGESQHLTRG-QLKE-YIGQQR-GEGYVFYEHRAY-GVA-G
   310            320            330            340            350


  640            650            660            670        680       690
NKAYIE---WTDP-ITKAKINTIPTSAEFIKHLSSIRRSSHVGVYK-DSGDKDEFAKKES
 •+  +        •  ++        •   •   +++••+•  +•  + ••6: •       •
-KSLFDDGLGAAPGVPSGRSKFSPDVLETVPASPGLRRPSLGAVERQDSG-YDSLDGVGS
   360            370            380            390        400       410


        700            710            720            730            740        750
VKKIAGYLSDYYNSANHIFSQEKKRKISIFRGIQAYNEIENVLKSKQIAPEYKNYFQYLK
 • +***                          ++   +  +  +  +
RSFSLGEVSDHAAVEAAELEHTRQVLHAGARQDDAEPGVSGASAHHGQRALQGAQAVAAA
       420            430            440            450            460        470


        760            770            780            790
ERITNQYQLLLTHQKSNIEFKLLYKQLNFTENETDHFEVFQKIIDEK
 +•*+      ++   ++      •      +       +
QRLVHAIALMTQFGRAGSTNTPQEAASLSAAVFGLGEASSAVAETVSGFFRGSSRHAGGF
       480            490            500            510            520        530
```

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | GENE, vol. 64, no. 2, 1988, pages 277-284, Elsevier Science Publishers B.V. (Biomedical Division), Amsterdam, NL; M. MOCK et al.: "Cloning and expression of the calmodulin-sensitive Bacillus anthracis adenylate cyclase in Escherica coli" * En entier * --- | 1-4,9, 13 | C 12 N 15/31 C 12 N 9/88 A 61 K 39/07 C 12 Q 1/68 A 61 K 39/10 A 61 K 39/40 C 12 P 21/08 |
| X | THE FASEB JOURNAL, vol. 2, no. 6, 25 mars 1988, résumé no. 8451; D. ROBERTSON et al.: "Biochemical analysis of the Bacillus anthracis edema factor gene: a calmodulin-dependent adenylate cyclase" * Résumé * --- | 1,2,9 | |
| D,X | JOURNAL OF BACTERIOLOGY, vol. 170, no. 5, mai 1988, pages 2263-2266, American Society for Microbiology; M.T. TIPPETTS et al.: "Molecular cloning and expression of the Bacillus anthracis edema factor toxin gene: a calmodulin-dependent adenylate cyclase" * En entier * --- | 1,2,4,9 | |
| A | FR-A-2 606 789 (INSTITUT PASTEUR) * Revendications 1,22,23 * --- | 14 | **DOMAINES TECHNIQUES RECHERCHES (Int. Cl.5)** C 12 N A 61 K |
| P,X | GENE, vol. 71, no. 2, novembre 1988, pages 293-298, Elsevier Science Publishers B.V. (Biomedical Division), Amsterdam, NL; V. ESCUYER et al.: "Structural homology between virulence-associated bacterial adenylate cyclases" * En entier * --- -/- | 1-13 | |

Le présent rapport a été établi pour toutes les revendications

| Lieu de la recherche | Date d'achèvement de la recherche | Examinateur |
|---|---|---|
| LA HAYE | 08-01-1990 | HUBER-MACK A. |

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | GENE, vol. 73, no. 2, décembre 1988, pages 363-371, Elsevier Science Publishers B.V. (Biomedical Division), Amsterdam, NL; D.L. ROBERTSON et al.: "Nucleotide sequence of the bacillus anthracis edema factor gene (cya): a calmodulin-dependent adenylate cyclase" * En entier * ----- | 1-13 | |
| | | | **DOMAINES TECHNIQUES RECHERCHES (Int. Cl.5)** |

Le présent rapport a été établi pour toutes les revendications

| Lieu de la recherche | Date d'achèvement de la recherche | Examinateur |
|---|---|---|
| LA HAYE | 08-01-1990 | HUBER-MACK A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&: membre de la même famille, document correspondant

EPO FORM 1503 03.82 (P0402)